(19) Europäisches Patentamt
European Patent Office
Office européen des brevets

(11) Publication number: **0 281 067 B1**

(12) **EUROPEAN PATENT SPECIFICATION**

(45) Date of publication of patent specification: **29.04.92** (51) Int. Cl.5: **C07H 13/04**, C07H 7/027

(21) Application number: **88103065.4**

(22) Date of filing: **01.03.88**

(54) **N-acetyl-3-fluoro-neuraminic acid derivatives and preparation thereof.**

(30) Priority: **06.03.87 JP 52358/87**

(43) Date of publication of application:
**07.09.88 Bulletin 88/36**

(45) Publication of the grant of the patent:
**29.04.92 Bulletin 92/18**

(84) Designated Contracting States:
**AT BE CH DE ES FR GB GR IT LI LU NL SE**

(56) References cited:
**EP-A- 0 177 783**
**FR-A- 2 506 313**

**CHEMISTRY LETTERS, 1986, pages 1449-1452, The Chemical Society of Japan; K. OKAMOTO et al.: "A stereospecific synthesis of beta-glycosides of N-Acetylneuraminic acid and secondary alcohols"**

**INTERNATIONAL JOURNAL OF APPLIED RADIATION AND ISOTOPES, vol. 36, no. 2, February 1985, pages 111-115, Pergamon Press Ltd, Oxford, GB; C.J.S. VAN RIJN et al.: "On the stereoselectivity of the reaction of [18F]Acetylhypofluorite with glucals"**

**CARBOHYDRATE RESEARCH, vol. 127, 1984, pages 201-210, Elsevier Science Publishers B.V., Amsterdam, NL; M.N. SHARMA et al.: "Synthesis and conformational studies of 2-beta-chloro, 2-alpha-fluoro, and 2-beta-fluoro deriatives of 2-deoxy-n-acetyl-neuraminic acid"**

(73) Proprietor: **MECT CORPORATION**
**1-1, Nishishinjuku 2-Chome**
**Shinjuku-ku Tokyo 163(JP)**

(72) Inventor: **Ohrui, Hiroshi**
**5-2-901, Odawara-Yamamotocho**
**Sendai-shi Miyagi(JP)**
Inventor: **Meguro, Hiroshi**
**1-14-3, Tsurugaya**
**Sendai-shi Miyagi(JP)**
Inventor: **Ido, Tatsuo**
**3-104, Kawauchi-Jyutaku Daiichi-chiku, Kawauchi**
**Sendai-shi Miyagi(JP)**

(74) Representative: **Leyh, Hans, Dr.-Ing. et al**
**Patentanwälte Berendt, Leyh & Hering Innere Wiener Strasse 20**
**W-8000 München 80(DE)**

**EP 0 281 067 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### BACKGROUND OF THE INVENTION

(1) FIELD OF THE INVENTION

This invention relates to novel derivatives of N-acetyl-3-fluoro-neuraminic acid and preparation thereof.

(2) RELATED ART STATEMENT

It has been known that a neuraminic derivative such as an N-acetylneuraminic acid, i.e. a sialic acid derivative, has been present in the animal kingdom or on a cell surface of some bacteria as a sialo-complex (glycoprotein, glycolipid, oligosaccharide and polysaccharide).

In recent years, the above sialic acid derivative is a compound regarded important in medical and pharmaceutical fields such as nervous function, cancer, inflamation, immunity, virus infection, differentiation and hormone receptor, and has attracted attention as a peculiar active molecule located on the cell surface.

However, as to the function of the sialic acid derivative acting in the aforesaid sialic acid complex, while there have been proposed many theories, uncertain points still remain and it is the present state that they are still a matter of conjecture.

Accordingly, in order to analyse its role, it has been regarded to be important to synthesize the sialic acid derivatives.

N-acetyl derivatives of a neuraminic acid which becomes a basic structure of the above sialic acid can be obtained by natural materials. As to fluorinated derivatives thereof, there have been known those in which fluorine is introduced at a 2-position [M.N. Shana and R. Eby, Carbohydr, Res., Vol. 127, p. 201 (1984)] or those introduced at a 9-position [M. Sharma and W. Korytnyl, J. Carbohydr. Chem., Vo. 1, p. 331 (1982 - 1983)].

### SUMMARY OF THE INVENTION

An object of the present invention is to provide N-acetyl-3-fluoro-neuraminicacid derivatives represented by the formula (I):

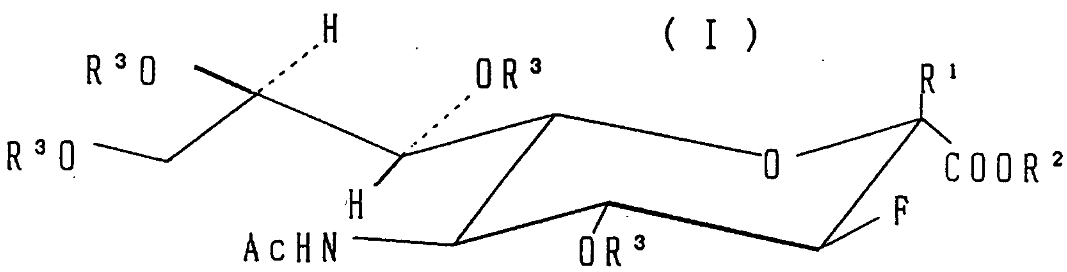

(I)

(wherein $R^1$ represents a fluorine atom, a hydroxyl group or $-OCOCH_3$; $R^2$ represents hydrogen or a lower alkyl group; $R^3$ represents hydrogen or an acetyl group, respectively).

Also, other object of the present invention is to provide an available preparative method of the N-acetyl-3-fluoro-neuraminic acid derivatives represented by the above formula (I).

The above and other objects and novel characteristics of the present invention will become more clear by the following detailed description and examples.

DETAILED DESCRIPTION OF THE PREFERRED EMBODIMENTS

The above N-acetyl-3-fluoro-neuraminic acid derivatives can be synthesized by passing, in an acetic acid solution or dichloromethane solution of alkyl 5-acetamido-2,6-anhydro-4,7,8,9-tetra-O-acetyl-2,3,5-trideoxy-D-glycero-D-galacto-2-enonate represented by the following formula (II):

(II)

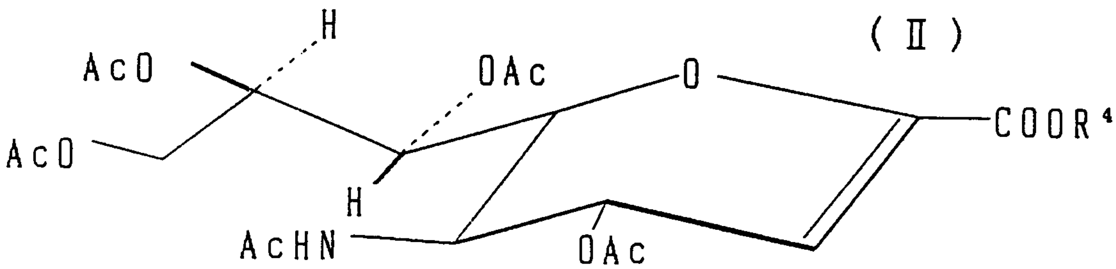

(wherein $R^4$ represents a lower alkyl group and Ac represents acetyl group, respectively),
either one of (1) a mixture of inert gas with $F_2$ or (2) acetylhypofluorite obtained by passing the mixture of inert gas with $F_2$ through a mixture of acetic acid/acetate, such as potassium acetate, therethrough, to carry out an addition reaction.
The above compound represented by the formula (II) can be obtained by, for example, the method as disclosed in [The 9th Saccharide Symposium Lecture Summary, p. B11 (1986)].

As an inert gas containing fluorine, argon containing 1 to 2 % of fluorine can be employed, and when this is passed through an acetic acid solution or dichloromethane solution of the compound represented by the above formula (II) at a lower temperature of 7 °C or so for 1 to 2 hours, a difluorinated material in which fluorine atoms are added at 2- and 3-position with cis-configuration as main product. At this time, two kinds of monofluorinated materials in which $-OCOCH_3$ and fluorine atom are added at 2-position and 3-position, respectively, with cis-configuration and trans-configuration, and formed simultaneously.

In order to separate and purify the product from the above reaction mixture, after evaporation of the solvent under reduced pressure, the residue is developed with a column chromatography used Silica gel, eluted with ethyl acetate/ether mixed solution, etc., and then recrystallization is carried out. Molecular structures including stereo-structure of each product are determined by $^1H$, $^{13}C$ and $^{19}F$ Nuclear Magnetic Resonance Spectra and optical rotation.

Also, the above addition reaction may be carried out by using acetylhypofluorite.

This acetylhypofluorite shall be used those which is prepared by passing argon containing 1 to 2 % of fluorine through a cartridge comprising a mixture of acetate such as potassium acetate, and acetic acid. When this is passed through an acetic acid aqueous solution or a dichloromethane solution of the compound represented by the above formula (II) at room temperature for 1 to 2 hours, monofluorinated material in which $-OCOCH_3$ and fluorine atom are added at 2-position and 3-position, respectively, with cis-configuration can be obtained as a main product.

At this time, a difluorinated material in which fluorine atoms are bonded at 2- and 3-positions with cis-configuration is formed at the same time. It can be estimated that this difluorinated material is formed by addition of unreacted fluorine passed through a cartridge comprising the above mixture of acetate and acetic acid.

Separation and purification of the product from the above reaction mixture is carried out by using the aforesaid silica gel column chromatography method.

Molecular structures including stereostructure of the monofluorinated material are determined by $^1H$, $^{13}C$, and $^{19}F$ Nuclear Magnetic Resonance Spectra and optical rotation.

Next, by deacetylation of the above difluorinated material with the method shown in the following Examples in detail, N-acetyl-2,3-difluoro-neuraminic acid ester can be obtained, and further by deesterification thereof, N-acetyl-2,3-difluoro-neuraminic acid can be obtained.

In the same manner, by deacetylation of the above monofluorinated material, N-acetyl-3-fluoro-neuraminic acid ester can be obtained, and further by deesterification thereof, N-acetyl-3-fluoro-neuraminic acid can be obtained.

According to the method of this invention, by using alkyl 5-acetamido-2;6-anhydro-4,7,8,9-tetra-O-acetyl-2,3,5-trideoxy-D-glycero-D-galacto-2-enoate as a starting material, and this is reacted with fluorine or acetylhypofluorite, novel derivatives of N-acetyl-3-fluoro-neuraminic acid incorporated fluorine atom stereoselectively with short time and good yield can be obtained.

These derivatives are not only useful for solving the action and the mechanism of sialic acid in the living body but also expected to be utilized as antiviral agents, as contraceptive pills, as immune adjustors or the like.

In the following, the present invention will be described in detail by referring Examples, but the present invention is not limited by these Examples.

Example 1

Into 2.3 g (4.8 mmole) of methyl 5-acetamido-2,6-anhydro-4,7,8,9-tetra-O-acetyl-2,3,5-trideoxy-D-glycero-D-galacto-2-enonate represented by the following formula (IIa):

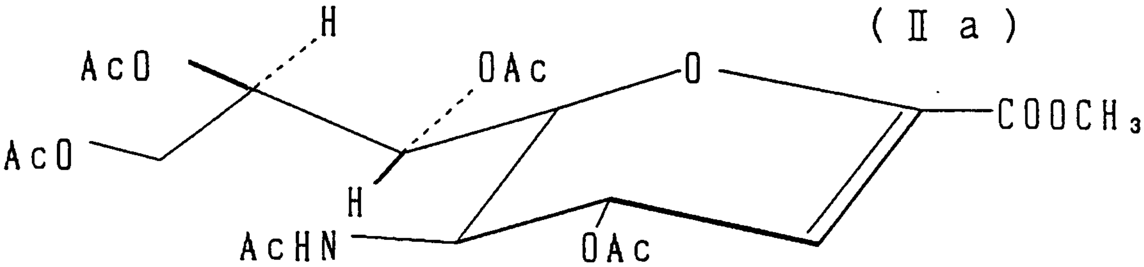

(II a)

dissolved in 25 ml of acetic acid solution (7 °C) was passed argon gas containing 1.7 % (6.1 mmole) of $F_2$ for 2 hours, and thereafter the solvent was distilled under reduced pressure to obtain 2.5 g of syrup. This syrup was developed to a column chromatography of Silica gel (available from Merck Co., "G 60", 250 g), eluted with a mixed solvent comprising ethyl acetate/ether = 3/1 (v/v) and recrystallized from a mixed solvent comprising dichloromethane/isopropyl ether to obtain the following three kinds of products.

(1) Methyl 5-acetamido-4,7,8,9-tetra-O-acetyl-2,3,5-trideoxy-2,3-difluoro-D-*erythro*-β-L-gluco-2-nonulosonate

[Molecular structure]

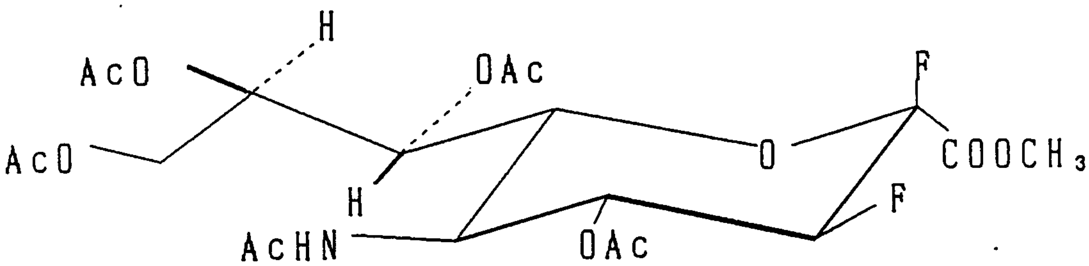

(Yielded amount: 900 mg, Yield: 36 %)

[Physical properties]

Melting point: 92 °C
$[\alpha]_D^{20}$ -48.12° (C = 0.55, chloroform)
Elemental analysis $C_{20}H_{27}N_1O_{12}F_2$
Calculated C : 46.97; H : 5.32; N : 2.73.
Observed C : 47.00; H : 5.37; N : 3.00.
$^1$H-NMR δ ($CDCl_3$)
5.00 (H-3, $J_{3,4}$ = 9.85 Hz)
5.39 (H-4, $J_{4,5}$ = 10.26 Hz)
4.40 (H-5, $J_{5,6}$ = 10.66 Hz)
4.27 (H-6, $J_{6,7}$ = 2.38 Hz)
5.39 (H-7, $J_{7,8}$ = 8.06 Hz)
5.16 (H-8, $J_{8,9}$ = 5.68 Hz, $J_{8,9'}$ = 2.71 Hz)
4.01, 4.32 (H-9, H-9', $J_{9,9'}$ = 12.55 Hz)

5.49 NH, $J_{NH,5}$ = 10.20 Hz)
3.91 (COOCH$_3$)
1.91 (NCOCH$_3$)
2.05, 2.10, 2.11, 2.13 (OAc).

(2) Methyl 5-acetamido-2,4,7,8,9-penta-O-acetyl-3,5-dideoxy-3-fluoro-D-*erythro*-β-gluco-2-nonulosonate

[Molecular structure]

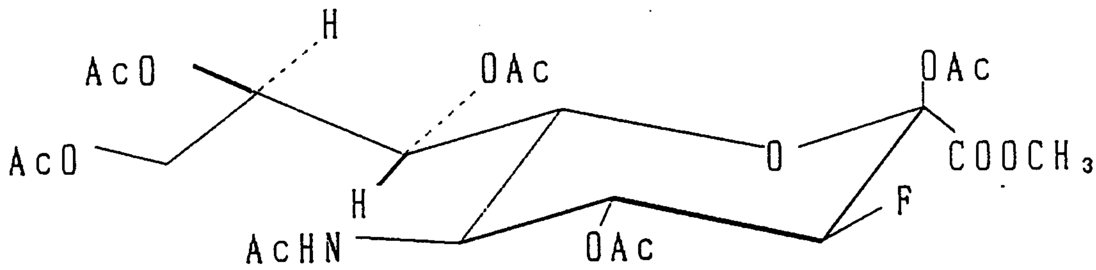

(Yielded amount: 190 mg, Yield: 7 %)

[Physical properties]

Melting point: 119 °C
$[\alpha]_D^{20}$ -56.5° (C = 0.21, $CHCl_3$)
Elemental analysis $C_{22}H_{30}NO_{14}F$
Calculated C : 47.91; H : 5.48; N : 2.53.
Observed C : 48.10; H : 5.35; N : 2.30.
$^1$H-NMR δ ($CDCl_3$)
4.62 (H-3, $J_{3,4}$ = 9.60 Hz)
5.48 (H-4, $J_{4,5}$ = 10.05 Hz)
4.24 (H-5, $J_{5,6}$ = 10.54 Hz)
4.11 (H-6, $J_{6,7}$ = 2.29 Hz)
5.35 (H-7, $J_{7,8}$ = 4.66 Hz)
5.02 (H-8, $J_{8,9}$ = 6.69 Hz, $J_{8,9'}$ = 2.44Hz)
4.14, 4.51 (H-9, H-9', $J_{9,9'}$ = 12.46 Hz)
5.58 (NH, $J_{NH,5}$ = 10.08 Hz)
3.85 (COOCH$_3$)
1.89 (NCOCH$_3$)
2.04, 2.06, 2.10, 2.15, 2.24 (OAc).

(3) Methyl 5-acetamido-2,4,7,8,9-penta-O-acetyl-3,5-dideoxy-3-fluoro-D-*erythro*-β-L-manno-2-nonulosonate

[Molecular structure]

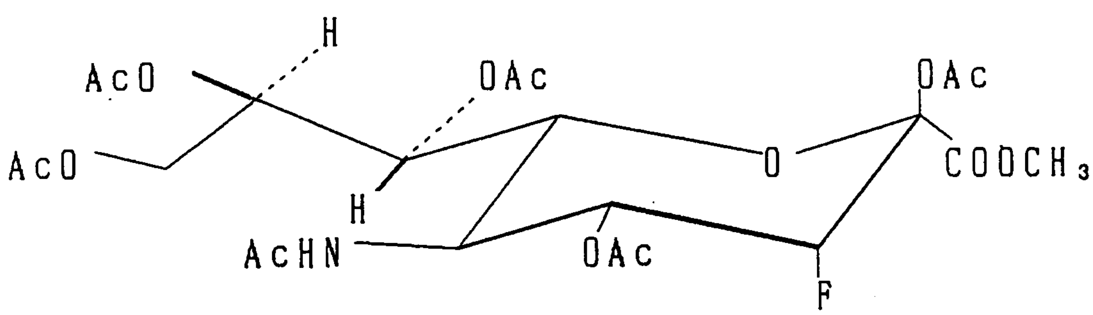

(Yielded amount: 30 mg, Yield: 7 %)

[Physical properties]

Melting point: 194 °C
$[\alpha]_D^{20}$ -24.5° (C = 0.19, chloroform)
Elemental analysis $C_{22}H_{30}NO_{14}F$
Calculated C : 47.91; H : 5.48; N : 2.53.
Observed C : 47.85; H : 5.53; N : 2.55.
$^1$H-NMR δ ($CDCl_3$)
4.94 (H-3, $J_{3,4}$ = 2.57 Hz)
5.50 (H-4, $J_{4,5}$ = 10.68 Hz)
4.14 (H-5, $J_{5,6}$ = 10.61 Hz)
4.25 (H-6, $J_{6,7}$ = 1.84 Hz)
5.35 (H-7, $J_{7,8}$ = 5.13 Hz)
5.13 (H-8, $J_{8,9}$ = 6.60 Hz, $J_{8,9'}$ = 2.20 Hz)
4.57 (H-9, H-9′, $J_{9,9'}$ = 12.46 Hz)
5.43 (NH, $J_{NH,5}$ = 8.79 Hz)
3.84 ($C\overline{O}OC\overline{H}_3$)
1.92 ($NCOC\overline{H}_3$)
2.04, 2.05, 2.11, 2.17, 2.18 (OAc).

Example 2

Acetylhypofluorite (AcOF) was prepared by passing an argon gas containing 1.5 % (10 mmole) of $F_2$ through a cartridge comprising a mixture of potassium acetate/acetic acid. This was passed through 480 mg (1.0 mmole) of methyl 5-acetamido-2,6-anhydro-4,7,8,9-tetra-O-acetyl-2,3,5-trideoxy-D-glycero-D-galacto-2-enonate used in Example 1 dissolved in 10 ml acetic acid solution at room temperature for an hour and then the solvent was distilled under reduced pressure at 60 °C to obtain 520 mg of syrup.

This syrup was developed with a column chromatography of Silica gel (available from Merck Co., "G 60", 60 g), eluted with a mixed solution comprising ethyl acetate/ether = 3/1 (v/v) and recrystallized from a mixed solution of dichloromethane/isopropyl ether to obtain the same products as in the above Example 1:

(1) Methyl 5-acetamido-4,7,8,9-tetra-O-acetyl-2,3,5-trideoxy-2,3-difluoro-D-*erythro*-β-L-gluco-2-nonulosonate (Yielded amount = 40 mg, Yield = 7.7 %) and

(2) Methyl 5-acetamido-2,4,7,8,9-penta-O-acetyl-3,5-dideoxy-3-fluoro-D-*erythro*-β-L-gluco-2-nonulosonate (Yielded amount = 190 mg, Yield = 34 %) can be obtained.

Example 3

To an anhydrous methanol solution (3 ml) containing 50 mg (0.098 mmole) of (1) methyl 5-acetamido-4,7,8,9-tetra-O-acetyl-2,3,5-trideoxy-2,3-difluoro-D-*erythro*-β-L-gluco-2-nonulosonate obtained in the aforesaid Example 1 or 2 was added 1 ml of methanol containing 2 mmole of sodium methoxide ($NaOCH_3$) and the mixture was stirred at room temperature for 30 minutes. To the reaction mixture was added a small excess amount of Dowex 50 [$H^+$] and the mixture was stirred. The resin was filtered and washed with 5 ml of methanol. The filtrate was condensed under reduced pressure and recrystallized from a mixed solvent of methanol/ethyl acetate/petroleum ether to obtain the following product (4) (Yielded amount = 26 mg, Yield = 77 %).

(4) Methyl 5-acetamido-2,3,5-trideoxy-2,3-difluoro-D-*erythro*-β-L-gluco-2-nonulosonate

[Molecular structure]

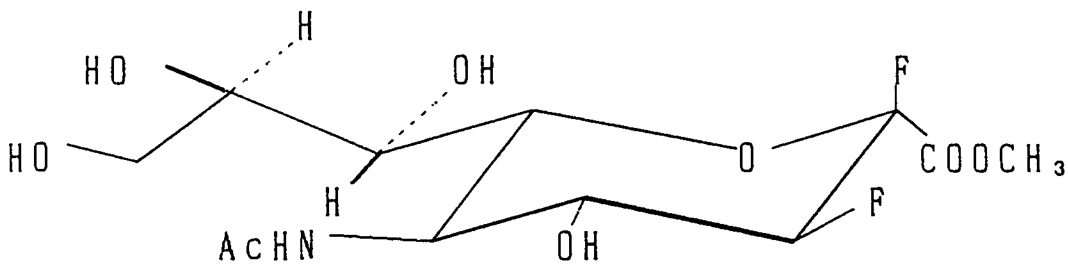

[Physical properties]

Melting point: 227 °C
$[\alpha]_D^{20}$ -49.79° (C = 1.45, $H_2O$)
Elemental analysis $C_{12}H_{19}NO_8F_2$
Calculated C : 41.98; H : 5.57; N : 4.08.
Observed C : 42.11; H : 5.63; N : 4.38.
$^1$H-NMR δ ($D_2O$)
4.88 (H-3, $J_{3,4}$ = 8.98 Hz)
4.18 - 4.26 (3H, H-4, H-5, H-6)
3.58 (H-7, $J_{6,7}$ = 0 Hz)
3.73 (H-8, $J_{7,8}$ = 9.16 Hz, $J_{8,9}$ = 3.30 Hz, $J_{8,9'}$ = 6.05 Hz)
3.63, 3.81 (H-9, H-9', $J_{9,9'}$ = 11.91 Hz)
2.06 ($NCOCH_3$)
3.95 ($COOC\underline{H}_3$)

Example 4

To 8 ml of anhydrous methanol solution containing 1 to 2 % of 0.5 N NaOH aqueous solution was added 250 mg (0.49 mmole) of methyl 5-acetamido-4,7,8,9-tetra-O-acetyl-2,3,5-trideoxy-2,3-difluoro-D-*erythro*-β-L-gluco-2-nonulosonate, and the mixture was stirred at room temperature for an hour.

To the reaction mixture was added 1 ml of methanol containing 2 mmole of sodium methoxide ($NaOCH_3$), and the mixture was stirred at room temperature for 30 minutes. To the reaction mixture was added a small excess amount of Dowex 50 [$H^+$] and the mixture was stirred. After filtration, the resin was washed with 10 ml of water. This filtrate was condensed under reduced pressure, and the residue was dissolved in water. A small amount of activated charcoal was added to the solution and the mixture was stirred. Thereafter, the activated charcoal was filtered off, and the filtrate was dried under vacuo to obtain the following product (5) (Yielded amount = 128 mg, Yield = 76 %).

(5) 5-Acetamido-2,3,5-trideoxy-2,3-difluoro-D-*erythro*-β-L-gluco-2- nonulosonic acid

[Molecular structure]

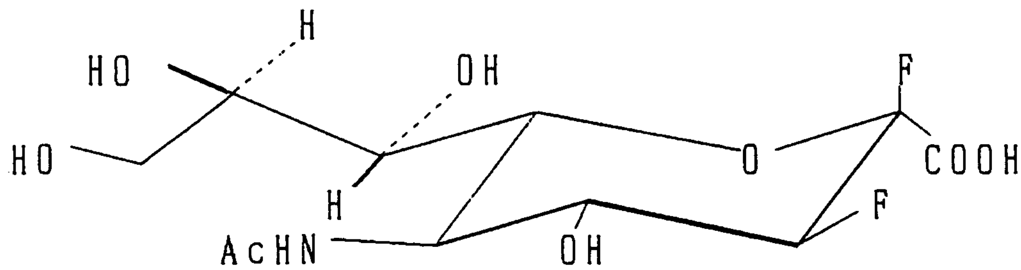

[Physical properties]

Melting point: 206 °C
$[\alpha]_D^{20}$ -45.2° (C = 2.22, $H_2O$)
Elemental analysis $C_{11}H_{17}NO_8F_2$
Calculated C : 40,12; H : 5.20; N : 4.25.
Observed C : 40.23; H : 5.11; N : 4.16.
$^1$H-NMR δ ($D_2O$)
4.80 (H-3, $J_{3,4}$ = 8.79 Hz)
4.12 - 4.23 (3H, H-4, H-5, H-6)
3.55 (H-7, $J_{6,7}$ = 0.74 Hz)
3.75 (H-8, $J_{7,8}$ = 8.66 Hz, $J_{8,9}$ = 5.87 Hz, $J_{8,9'}$ = 2.75 Hz)
3.63, (H-9, H-9′, $J_{9,9'}$ = 11.73 Hz) 3.82.
2.06 (NCOC$\underline{H}_3$).

Example 5

By using 55 mg of (2) methyl 5-acetamido-2,4,7,8,9-penta-O-acetyl-3,5-dideoxy-3-fluoro-D-*erythro*-β-L-gluco-2-nonulosonate obtained in the aforesaid Example 1 or 2, the following compound (6) was obtained in the same manner as in the above Example 3.

(6) Methyl 5-acetamido-3,5-dideoxy-3-fluoro-D-*erythro*-β-L-gluco-2-nonulosonate

[Physical properties]

Melting point: 209 °C (Recrystallized from a mixed solution of methanol/ethyl acetate/petroleum ether)
$[\alpha]_D^{20}$ -41.14° (C = 0.86, $H_2O$)
Elemental analysis $C_{12}H_{20}NO_9F$
Calculated C : 42.23; H : 5.9; N : 4.1.
Observed C : 42.37; H : 6.0; N : 4.3.
$^1$H-NMR δ ($D_2O$)
4.75 (H-3, $J_{3,4}$ = 8.98 Hz)
4.09 - 4.18 (3H, H-4, H-5, H-6)
3.56 (H-7, $J_{6,7}$ = 1.10 Hz)
3.71 (H-8, $J_{7,8}$ = 8.79 Hz, $J_{8,9}$ = 6.05 Hz, $J_{8,9'}$ = 3.43 Hz)
3.63, 3.8 (H-9, H-9′, $J_{9,9'}$ = 11.9 Hz)
2.06 (NCOC$H_3$)
3.91 (COOC$\underline{H}_3$).

Example 6

To 1 ml of methanol solution containing 20 % of 1N NaOH aqueous solution was added 20 mg (0.036 mmole) of (2) methyl 5-acetamido-2,4,7,8,9-penta-O-acetyl-3,5-dideoxy-3-fluoro-D-*erythro*-β-L-gluco-2-nonulosonate obtained in the aforesaid Example 1 or 2, and the mixture was stirred at room temperature for an hour.

Thereafter, by processing the same treatment as in the aforesaid Example 4, the following compound (7) can be obtained (Yielded amount = 9 mg, Yield = 76 %).

(7) 5-Acetamido-3,5-dideoxy-3-fluoro-D-*erythro*-β-L-gluco-2-nonulosonic acid

[Molecular structure]

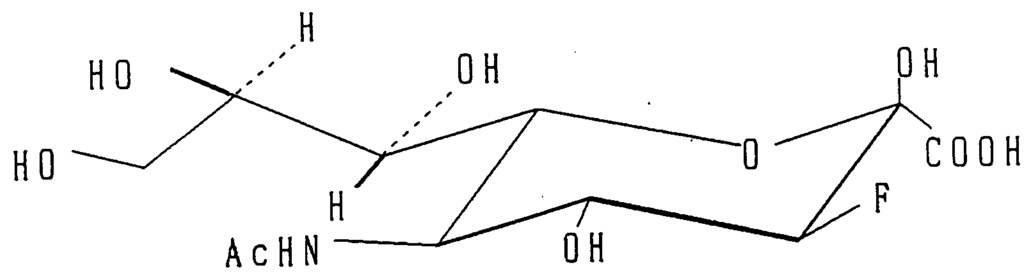

[Physical properties]

Melting point: 205 °C
$[\alpha]_D^{20}$ -40.02° (C = 0.86, $H_2O$)
Elemental analysis $C_{11}H_{18}NO_9F$
Calculated C : 40.37; H : 5.54; N : 4.27.
Observed C : 40.07; H : 5.51; N : 4.21.
$^1$H-NMR δ ($D_2O$)
4.71 (H-3, $J_{3,4}$ = 8.42 Hz)
4.10 - 4.15 (3H, H-4, H-5, H-6)
3.54 (H-7, $J_{6,7}$ = 0 Hz)
3.73 (H-8, $J_{7,8}$ = 8.79 Hz, $J_{8,9}$ = 6.05 Hz, $J_{8,9'}$ = 2.75 Hz)
3.63, 3.83 (H-9, H-9', $J_{9,9'}$ = 11.91 Hz).
2.06 (NCOC$\underline{H}_3$).

## Claims

**1.** A N-acetyl-3-fluoro-neuraminic acid derivative represented by the formula (I):

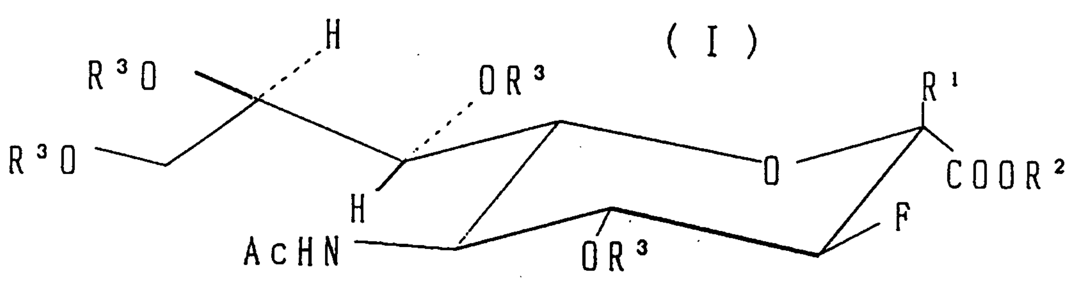

wherein $R^1$ represents a fluorine atom, a hydroxyl group or $-OCOCH_3$; $R^2$ represents hydrogen or a lower alkyl group; $R^3$ represents hydrogen or an acetyl group, respectively.

2. A method for preparing a N-acetyl-3-fluoro-neuraminic acid derivative represented by the formula (I):

(I)

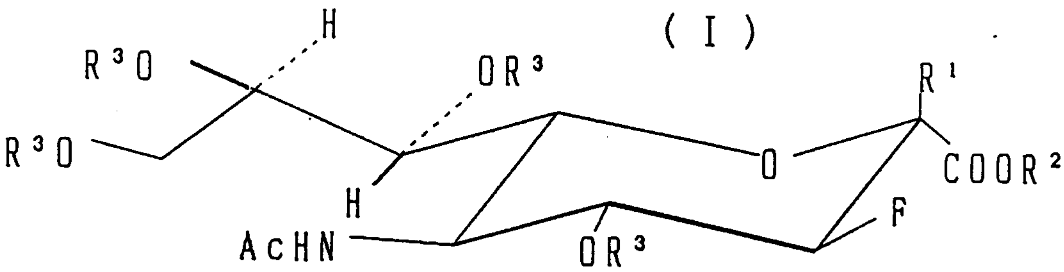

wherein $R^1$ represents a fluorine atom, a hydroxyl group or $-OCOCH_3$; $R^2$ represents hydrogen or a lower alkyl group; $R^3$ represents hydrogen or an acetyl group, respectively, which comprises reacting a solution of alkyl 5-acetamido-2,6-anhydro-4,7,8,9-tetra-O-acetyl-2,3,5-trideoxy-D-glycero-D-galacto-2-enonate represented by the formula (II):

(II)

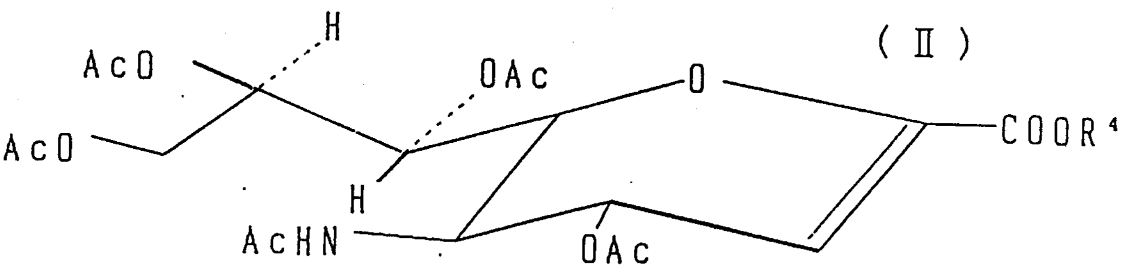

wherein $R^4$ represents a lower alkyl group and Ac represents an acetyl group, respectively with a mixture of inert gas with $F_2$ or acetylhypofluorite obtained by passing the mixture of inert gas with $F_2$ through a mixture of acetic acid/acetate by passing thereinto, and then removing the solvent and subjecting the residue to silica gel column chromatography to isolate the desired compound.

3. The method for preparing the N-acetyl-3-fluoro-neuraminic acid derivative according to Claim 2, wherein said compound is represented by the formula:

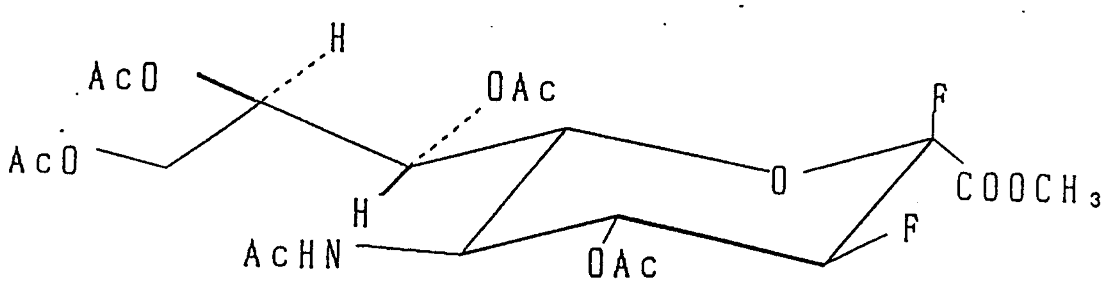

wherein Ac represents acetyl group.

4. The method for preparing the N-acetyl-3-fluoro-neuraminic acid derivative according to Claim 2, wherein said compound is represented by the formula:

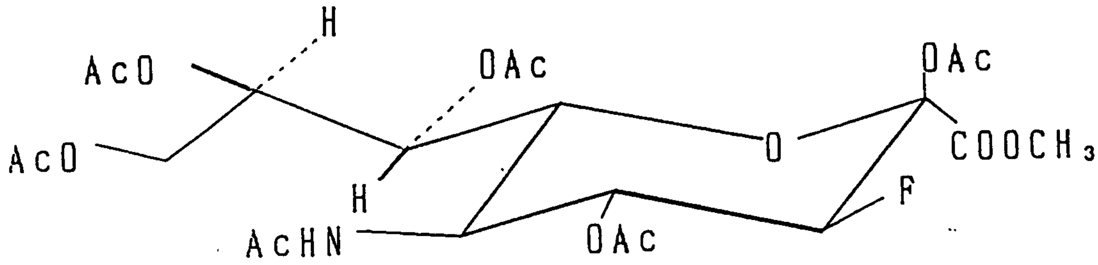

wherein Ac represents acetyl group.

5. The method for preparing the N-acetyl-3-fluoro-neuraminic acid derivative according to Claim 2, wherein said compound is represented by the formula:

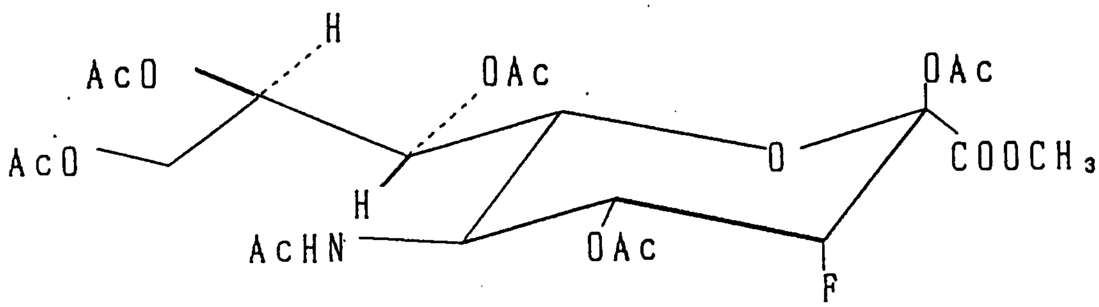

wherein Ac represents acetyl group.

6. The method for preparing the N-acetyl-3-fluoro-neuraminic acid derivative according to Claim 2, wherein said compound is represented by the formula:

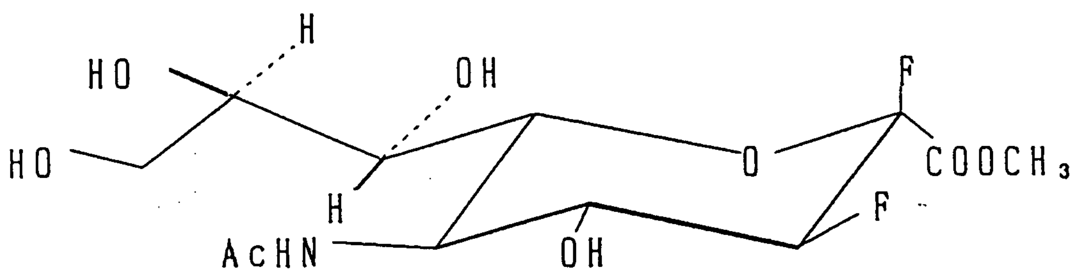

wherein Ac represents acetyl group.

7. The method for preparing the N-acetyl-3-fluoro-neuraminic acid derivative according to Claim 2, wherein said compound is represented by the formula:

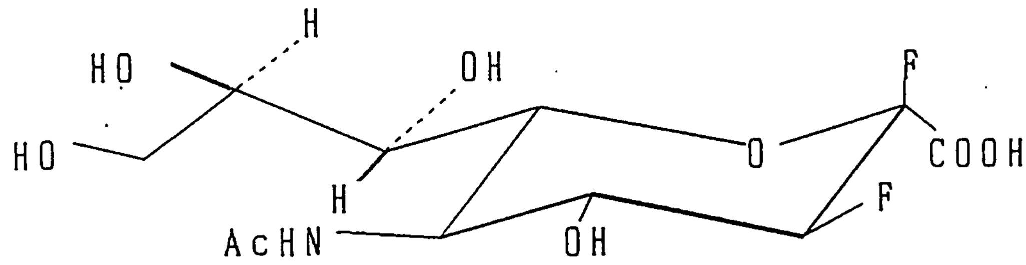

wherein Ac represents acetyl group.

8. The method for preparing the N-acetyl-3-fluoro-neuraminic acid derivative according to Claim 2, wherein said compound is represented by the formula:

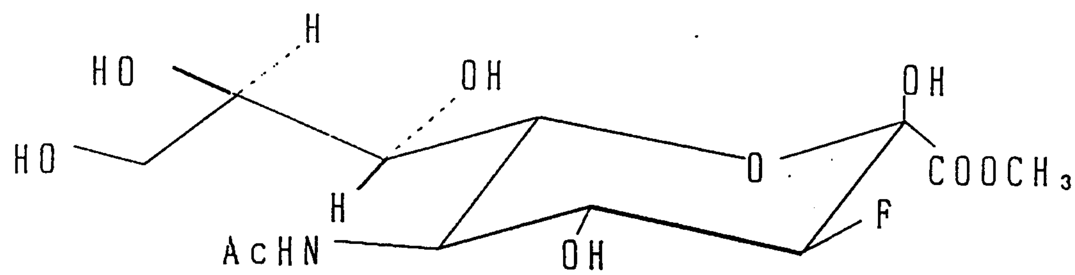

wherein Ac represents acetyl group.

9. The method for preparing the N-acetyl-3-fluoro-neuraminic acid derivative according to Claim 2, wherein said compound is represented by the formula:

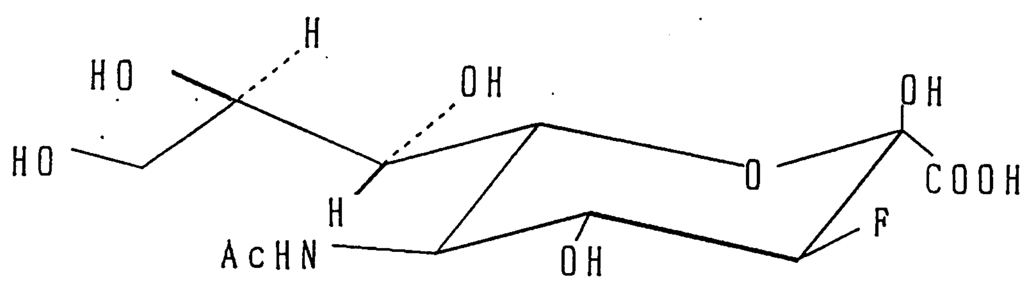

wherein Ac represents acetyl group.

## Revendications

1. Dérivé d'un acide N-acétyl-3-fluoro-neuraminique représenté par la formule (I) :

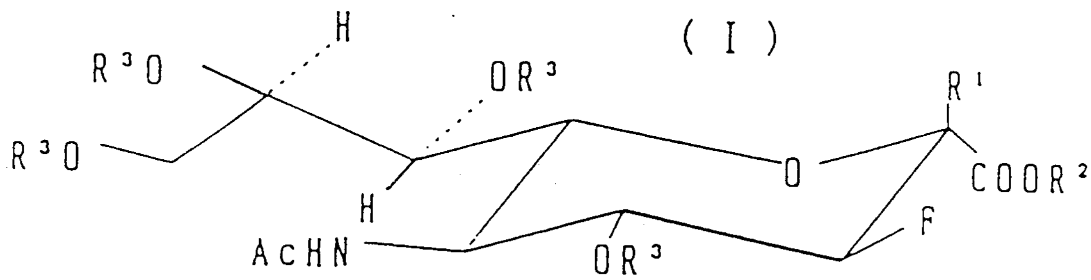

( I )

dans laquelle $R^1$ représente un atome de fluor, un groupe hydroxyle ou $-OCOCH_3$; $R^2$ représente un atome d'hydrogène ou un groupe alcoyle inférieur; $R^3$ représente un atome d'hydrogène ou un groupe acétyle, respectivement.

2. Procédé pour préparer un dérivé d'un acide N-acétyl-3-fluoro-neuraminique représenté par la formule (I) :

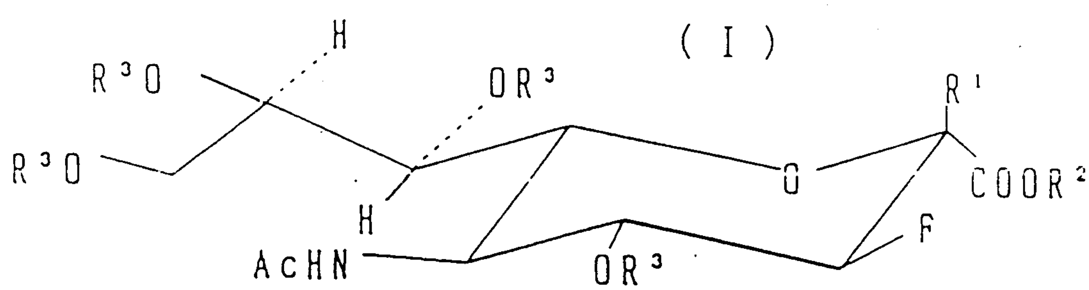

( I )

dans laquelle R1 représente un atome de fluor, un groupe hydroxyle ou $-OCOCH_3$; $R^2$ représente un atome d'hydrogène ou un groupe alcoyle inférieur, $R^3$ représente un atome d'hydrogène ou un groupe acétyle, respectivement, qui consiste à faire réagir une solution de 5-acétamido-2,6-anhydro-4,7,8,9-tétra-O-acétyl-2,3,5-tridéoxy-D-glycéro-D-galacto-2-énonate d'alcoyle représenté par la formule :

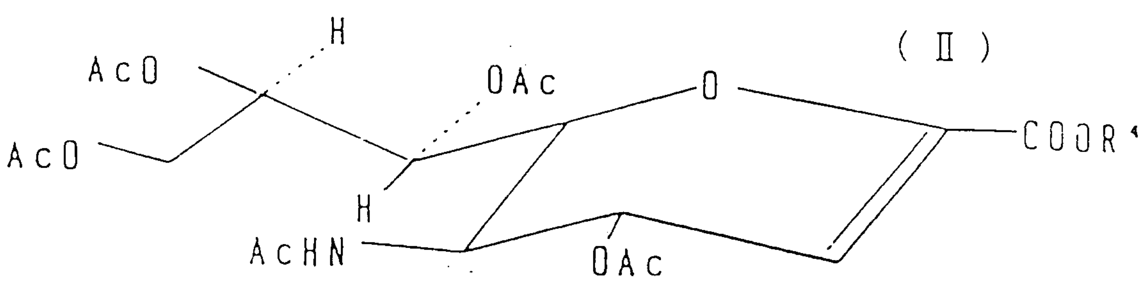

( II )

dans laquelle $R^4$ représente un groupe alcoyle inférieur et Ac représente un groupe acétyle, respectivement

avec un mélange d'un gaz inerte avec $F_2$ ou avec un hypofluorite d'acétyle obtenu en faisant passer le mélange du gaz inerte avec $F_2$ à travers un mélange d'acide acétique et d'acétate et en enlevant ensuite le solvant et en soumettant le résidu à une chromatographie sur colonne de gel de silice pour isoler le composé désiré.

3. Procédé pour préparer le dérivé de l'acide N-acétyl-3-fluoroneuraminique selon la revendication 2, dans lequel le composé est représenté par la formule :

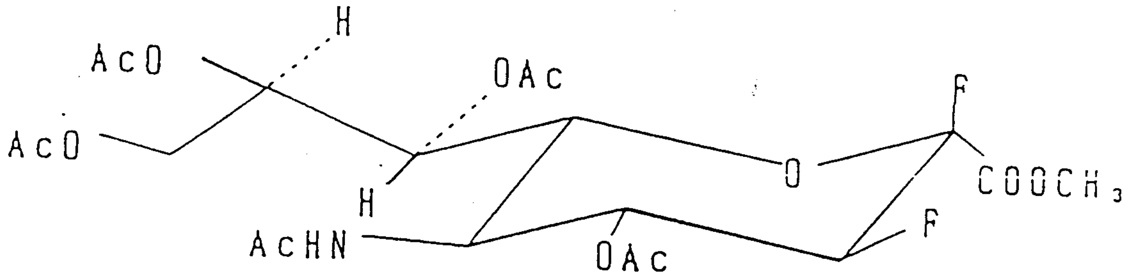

dans laquelle Ac représente un groupe acétyle.

4. Procédé pour préparer le dérivé de l'acide N-acétyl-3-fluoroneuraminique selon la revendication 2, dans lequel ce composé est représenté par la formule :

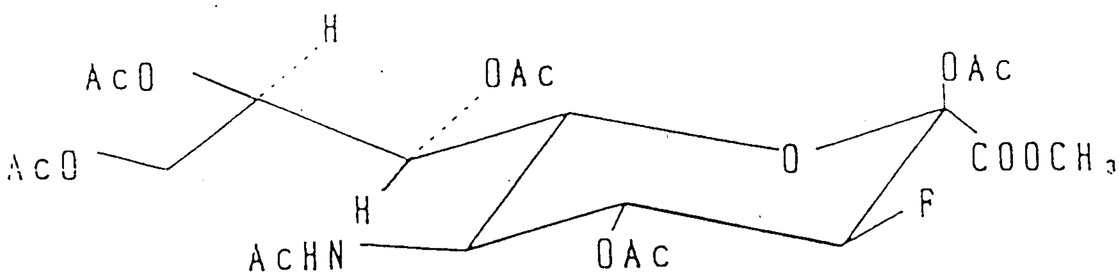

dans laquelle Ac représente un groupe acétyle.

5. Procédé pour préparer le dérivé de l'acide N-acétyl-3-fluoroneuraminique selon la revendication 2, dans lequel le composé est représenté par la formule :

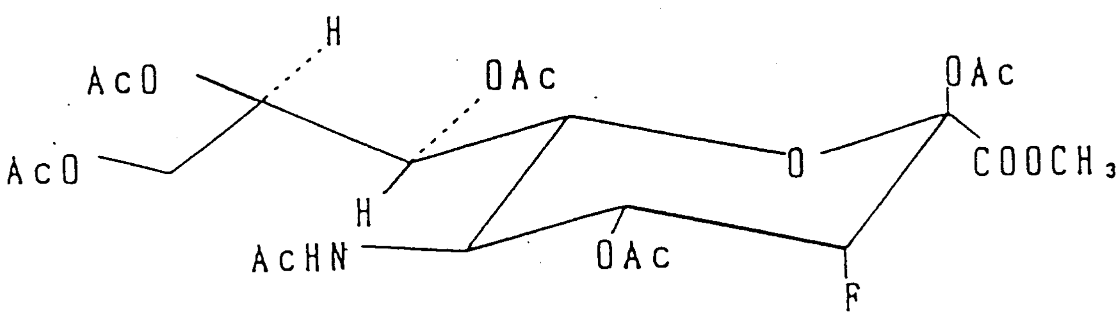

dans laquelle Ac représente un groupe acétyle.

6. Procédé pour préparer le dérivé de l'acide N-acétyl-3-fluoroneuraminique selon la revendication 2, dans lequel le composé est représenté par la formule :

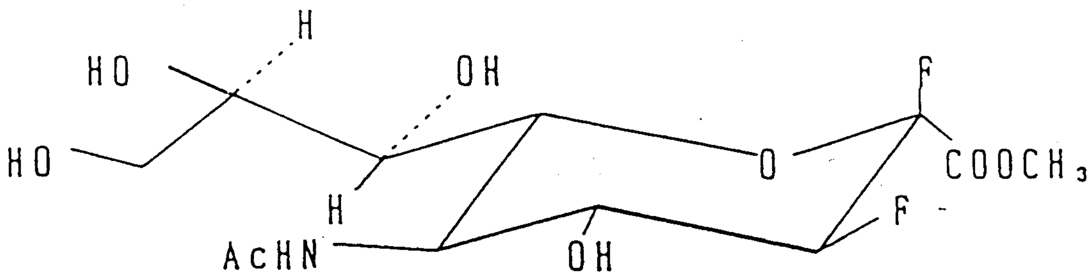

dans laquelle Ac représente un groupe acétyle.

7. Procédé pour préparer le dérivé de l'acide N-acétyl-3-fluoroneuraminique selon la revendication 2, dans lequel le composé est représenté par la formule :

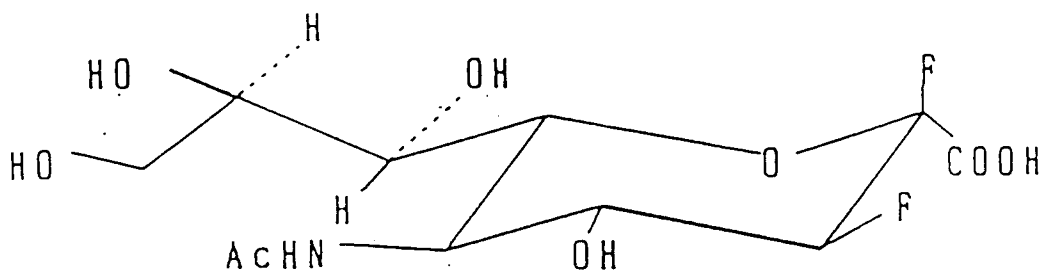

dans laquelle Ac représente un groupe acétyle.

8. Procédé pour préparer le dérivé de l'acide N-acétyl-3-fluoroneuraminique selon la revendication 2, dans lequel le composé est représenté par la formule :

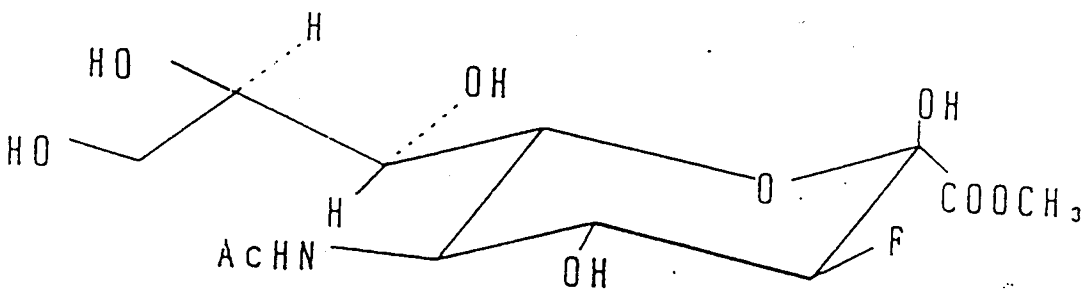

dans laquelle Ac représente un groupe acétyle.

9. Procédé pour préparer le dérivé de l'acide N-acétyl-3-fluoroneuraminique selon la revendication 2, dans lequel le composé est représenté par la formule :

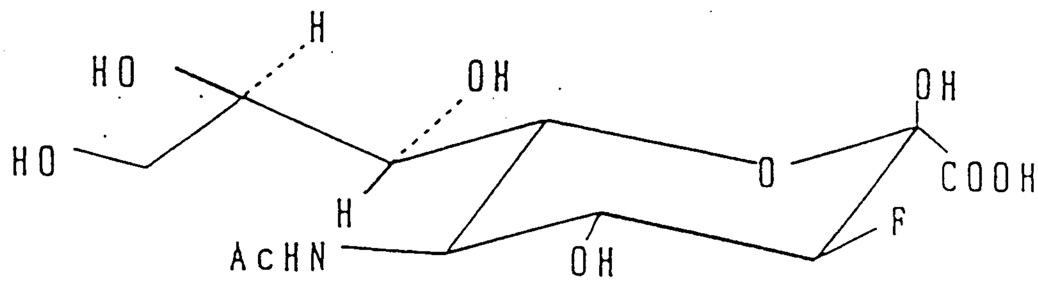

dans laquelle Ac représente un groupe acétyle.

**Patentansprüche**

1. N-acetyl-3-fluor-neuraminsäure-Derivat, dargestellt durch die Formel (I):

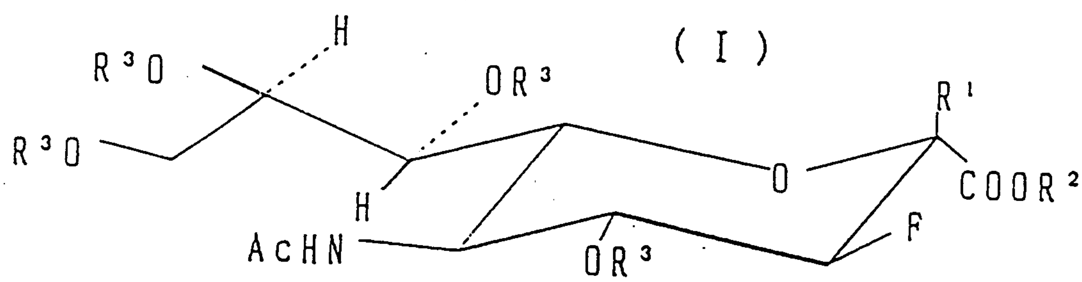

(I)

wobei $R^1$ ein Fluoratom, eine Hydroxylgruppe oder $-OCOCH_3$; $R^2$ Wasserstoff oder eine niedere Alkylgruppe; und $R^3$ Wasserstoff oder eine Acetylgruppe darstellen.

2. Verfahren zur Herstellung eines N-acetyl-3-fluor-neuraminsäure-Derivates, dargestellt durch die Formel (I):

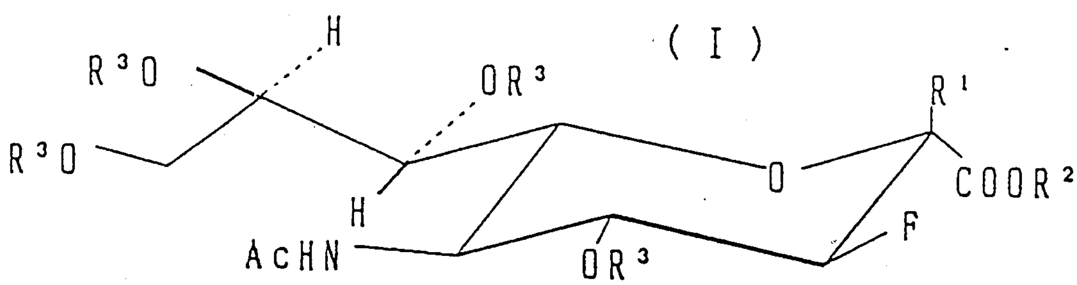

(I)

wobei $R^1$ ein Fluoratom, eine Hydroxylgruppe oder $-OCOCH_3$; $R^2$ Wasserstoff oder eine niedere Alkylgruppe; und $R^3$ Wasserstoff oder eine Acetylgruppe darstellen, das umfaßt, eine Lösung von Alkyl-5-acetamido-2,6-anhydro-4,7,8,9-tetra-O-acetyl-2,3,5-tridesoxy-D-glycero-D-galakto-2-enonat, dargestellt durch die Formel (II):

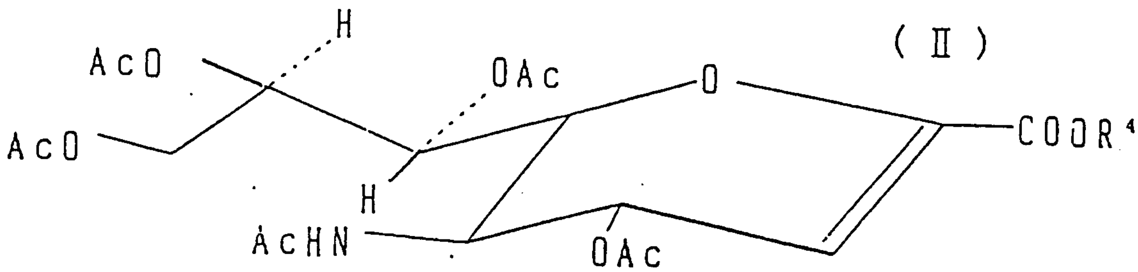

( II )

wobei $R^4$ eine niedere Alkylgruppe und Ac eine Acetylgruppe darstellen, mit einem Gemisch aus einem Inertgas mit $F_2$ oder Acetylhypofluorit, erhalten mittels Durchleiten des Gemisches aus Inertgas mit $F_2$ durch ein Gemisch aus Essigsäure/Acetat, umzusetzen, das Lösungsmittel zu entfernen und den Rückstand zur Isolation der gewünschten Verbindung einer Silicagel-Säulen-Chromatographiezu unterwerfen.

**3.** Verfahren zur Herstellung des N-acetyl-3-fluor-neuraminsäure-Derivates nach Anspruch 2, wobei die Verbindung durch die folgende Formel :

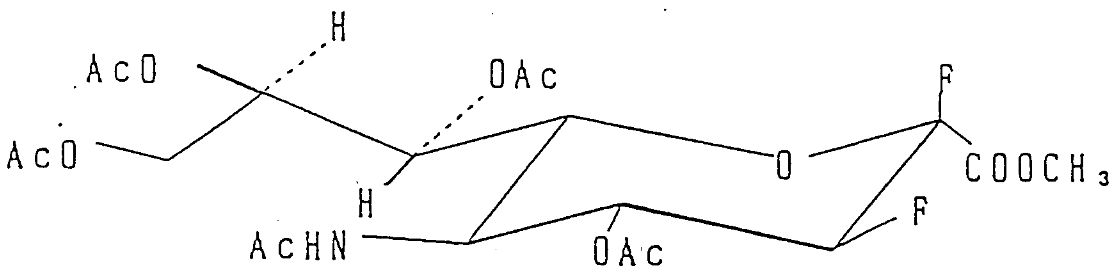

dargestellt wird, wobei Ac eine Acetylgruppe darstellt.

**4.** Verfahren zur Herstellung des N-acetyl-3-fluor-neuraminsäure-Derivats nach Anspruch 2, wobei die Verbindung durch die Formel:

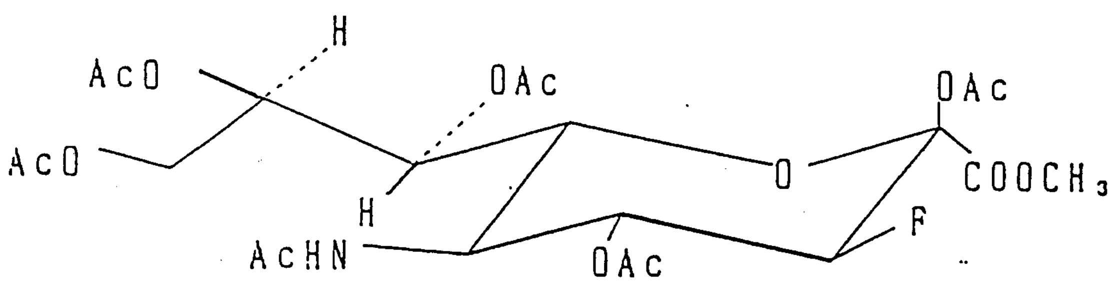

dargestellt wird, wobei Ac eine Acetylgruppe darstellt.

5. Verfahren zur Herstellung des N-acetyl-3-fluor-neuraminsäure-Derivates nach Anspruch 2, wobei die Verbindung durch die Formel:

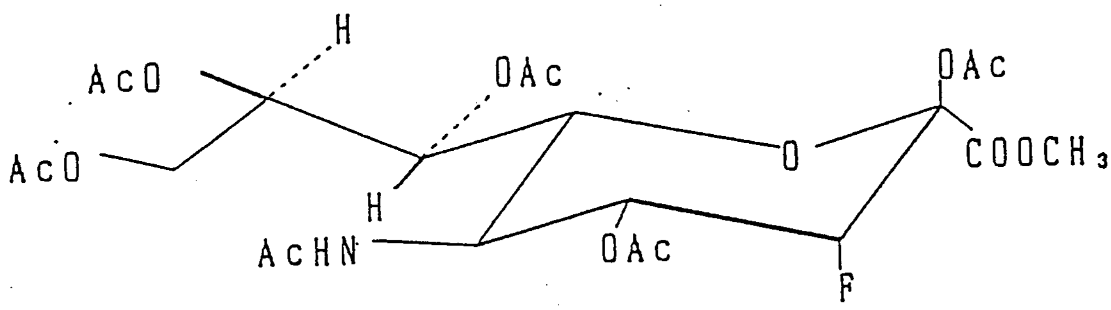

dargestellt wir, wobei Ac eine Acetylgruppe darstellt.

6. Verfahren zur Herstellung des N-acetyl-3-fluor-neuraminsäure-Derivates nach Anspruch 2, wobei die Verbindung durch die folgende Formel:

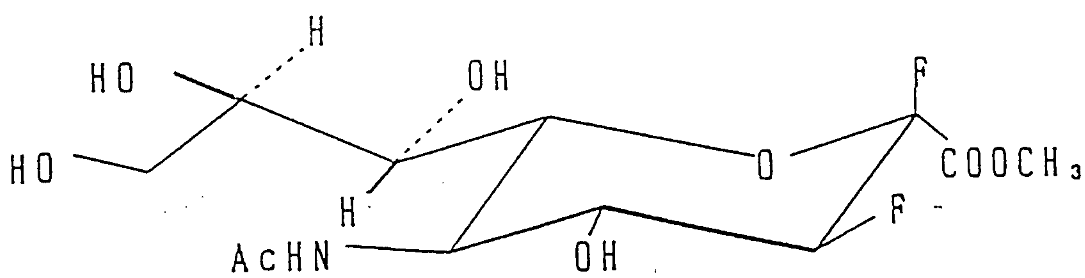

dargestellt wird, wobei Ac eine Acetylgruppe darstellt.

7. Verfahren zur Herstellung des N-acetyl-3-fluor-neuraminsäure-Derivats nach Anspruch 2, wobei die Verbindung durch die Formel:

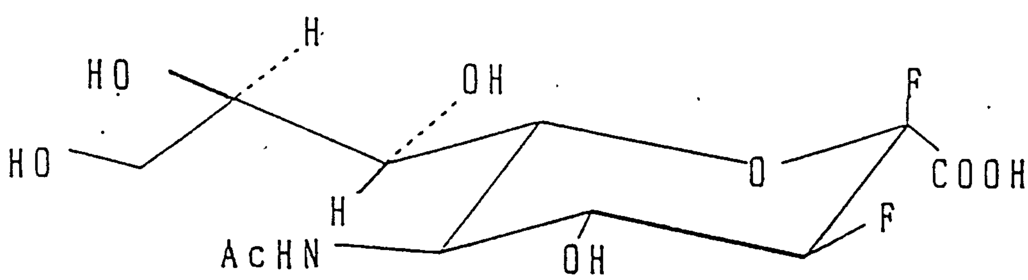

dargestellt wird, wobei Ac eine Acetylgruppe darstellt.

8. Verfahren zur Herstellung des N-acetyl-3-fluor-neuraminsäure-Derivats nach Anspruch 2, wobei die Verbindung durch die Formel:

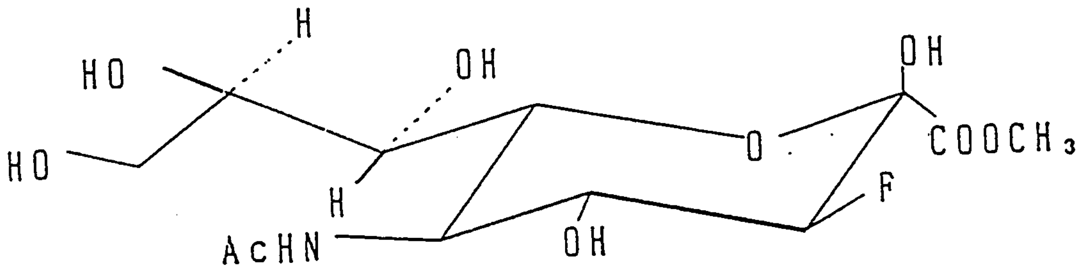

dargestellt wird, wobei Ac eine Acetylgruppe darstellt.

9. Verfahren zur Herstellung des N-acetyl-3-fluor-neuraminsäure-Derivates nach Anspruch 2, wobei die Verbindung durch die Formel:

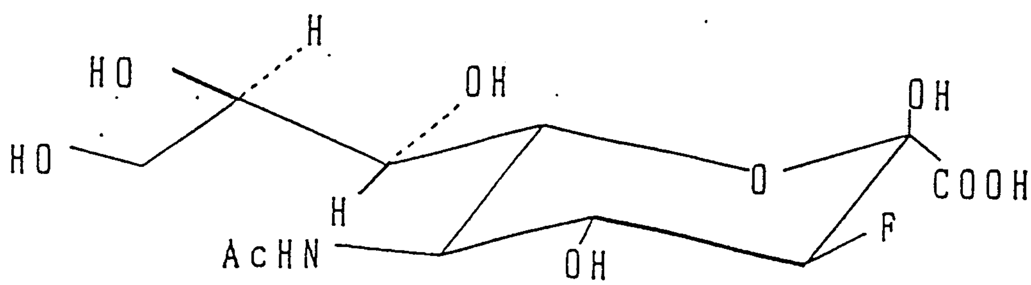

dargestellt wir, wobei Ac eine Acetylgruppe darstellt.